(19) Europäisches Patentamt
European Patent Office
Office européen des brevets

(11) Numéro de publication: **0 210 904 B1**

(12) **FASCICULE DE BREVET EUROPEEN**

(45) Date de publication du fascicule du brevet: **06.09.89**

(51) Int. Cl.⁴: **C 07 D 295/10**, A 61 K 31/445

(21) Numéro de dépôt: **86401499.8**

(22) Date de dépôt: **04.07.86**

(54) **Phényl-(3-hexaméthylèneiminopropyl)-cétone, procédé de préparation et utilisation en thérapeutique.**

(30) Priorité: **10.07.85 FR 8510551**

(43) Date de publication de la demande: **04.02.87 Bulletin 87/6**

(45) Mention de la délivrance du brevet: **06.09.89 Bulletin 89/36**

(84) Etats contractants désignés: **AT BE CH DE FR GB IT LI LU NL SE**

(56) Documents cités:
**EP-A- 0 110 748**
**DE-A- 1 916 055**
**GB-A- 682 161**
**US-A- 3 922 266**

(73) Titulaire: **LABORATOIRE L. LAFON Société anonyme dite:, 1 rue Georges Médéric, F-94701 Maisons-Alfort (FR)**

(72) Inventeur: **Lafon, Louis, 5, rue d'Alboni, F-75016 Paris (FR)**

(74) Mandataire: **Le Guen, Gérard et al, CABINET LAVOIX 2, place d'Estienne d'Orves, F-75441 Paris Cédex 09 (FR)**

EP 0 210 904 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne en tant que produits industriels nouveaux des dérivés de phényl-(3-aminopropyl)-cétone, à savoir la phényl-(3-hexaméthylèneiminopropyl)-cétone et ses sels d'addition. Elle concerne également le procédé de préparation et l'utilisation en thérapeutique de ces produits, notamment en tant qu'agents cardiovasculaires eu égard à leurs propriétés vasodilatatrices bénéfiques.

On sait que l'on a déjà décrit dans le passé un certain nombre de dérivés du type (phényl)-(aminoalkyl)-cétone où le groupe phényle est substitué par les groupes alkoxy et/ou hydroxy, voir à cet effet FR-A-1 492 256, FR-A-5636M, FR-A-2 404 003, FR-A-2 534 916, GB-A-1 078 975, GB-A-1 115 992, US-A-3 895 030 et l'article de A. Boucherle et al., Chimie Thérapeutique 3 (No. 4), 256-259, (1968), où lesdits dérivés ont été présentés en tant qu'agents antiinflammatoires, antalgiques, antipyrétiques, antispasmodiques, tranquillisants, vasodilatateurs et/ou bradycardisants.

On sait, notamment de FR-A-2 404 003, qu'il n'y a pas de relation structure-activité à l'intérieur de la famille des phényl-(aminoakyl)-cétones, les effets pharmacologiques étant modifiés ou disparaissant en fonction de la nature des substituants du groupe phényle, de la nature du groupe amino et enfin de la nature du groupe hydrocarboné aliphatique présent entre le groupe CO et le groupe amino.

On vient de trouver à présent que les composés selon l'invention, qui sont structurellement différents des composés antérieurement décrits ou suggérés, sont particulièrement intéressants en thérapeutique, notamment en raison de leurs propriétés vasodilatatrices améliorées et de leur toxicité plus faible par rapport aux produits connus les plus proches.

Les essais comparatifs consignés dans le tableau I ci-après montrent notamment que, par rapport (i) au chlorhydrate de (2,4,6-triméthoxyphényl)-(3-pyrrolidinopropyl)-cétone (No. de Code LL 1656), qui est décrit dans US-A-3 895 030 et commercialisé sous le nom de marque de «Fonzylane» (dénomination Commune Internationale : Chlorhydrate de Buflomedil) et qui est un agent vasodilatateur de référence, et (ii) au chlorhydrate de (2,4,6-triméthoxyphényl)-(3-hexaméthylèneiminopropyl)-cétone (No. de Code CRL 41 080), qui est décrit dans FR-A-2 534 916, le chlorhydrate de phényl-(3-hexaméthylèneiminopropyl)-cétone (No. de Code CRL 41 339) selon l'invention est plus actif en tant qui substance vasodilatatrice par voie intraduodénale, et, moins toxique.

Les nouveaux dérivés de phényl-aminoalkylcétone selon l'invention sont caractérisés en ce qu'ils sont choisis parmi l'ensemble constitué par

(a) la phényl-(3-hexaméthylèneiminopropyl)-cétone de formule

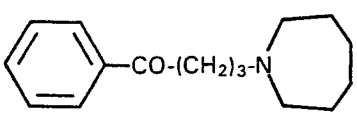

(I)

et

(b) ses sels d'addition.

Par sels d'addition, on entend ici les sels d'addition d'acide obtenus par réaction de la base libre de formule I avec un acide minéral ou organique, d'une part, et les sels d'ammonium, d'autre part. Parmi les acides utilisables pour salifier les bases libres de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulphonique, p-toluènesulfonique. Parmi les composés permettant d'obtenir des sels d'ammonium, on peut notamment citer $ICH_3$ et $ClCH_3$. D'une manière générale les sels d'addition d'acide sont préférés aux sels d'ammonium. Parmi les sels d'addition d'acide, le sel préféré est ici le chlorhydrate.

La base de formule I peut être préparée selon une méthode connue par application de mécanismes réactionnels classiques. Le procédé que l'on préconise ici consiste à faire réagir le 4-hexaméthylèneiminobutyronitrile avec le bromure de phénylmagnésium pendant au moins 1 h à une température comprise entre 0 et 25°C dans un solvant anhydre convenable, à raison de plus d'une mole (de préférence 2 moles) de $C_6H_5MgBr$ pour une mole de 4-hexaméthylèneimino-butyronitrile. Parmi les solvants qui conviennent on peut notamment citer les éthers tels que le diméthyléther, le diéthyléther, le tétrahydrofuranne et leurs mélanges.

La phényl-(3-hexaméthylèneiminopropyl)-cétone et ses sels sont des agents utiles dans le traitement des maladies du système cardiovasculaire; ils agissent en tant qu'agents vasodilatateurs périphériques et bradycardisants.

Selon l'invention, on préconise une composition thérapeutique qui est caractérisée en ce qu'elle renferme, en association avec un excipient physiologiquement acceptable, au moins un composé choisi parmi l'ensemble constitué pat la phényl-(3-hexaméthylèneiminopropyl)-cétone, ses sels d'addition non-toxiques et leurs mélanges.

Bien entendu dans une telle composition l'ingrédient actif intervient à une dose pharmaceutiquement efficace.

On préconise enfin selon l'invention l'utilisation d'une substance choisie parmi la phényl-(3-hexaméthylèneiminopropyl)-cétone et ses sels d'addition non-toxiques pour l'obtention d'un médicament vasodilatateur destiné à une utilisation thérapeutique pour les patients atteints de troubles cardiovasculaires (notamment des troubles circulatoires des membres inférieurs) et ayant besoin d'un tel médicament.

D'autres avantages et caractéristiques de l'invention seront mieux compris à la lecture qui va suivre (i) d'un exemple de préparation, et (ii) de résultats d'essais pharmacologiques, l'ensemble de ces éléments n'étant nullement limitatif mais donné à titre d'illustration.

*Préparation*

*Obtention du chlorhydrate de phényl-(3-hexaméthylèneiminopropyl)-cétone*

(No. de Code: CRL 41 339).

Autres nomenclatures: chlorhydrate de 1-benzoyl-3-hexaméthylèneimino-propane, ou chlorhydrate de (4-hexaméthylèneimino-1-butyryl)-benzène.

Au sein de 88 ml (0,2650 mole) d'une solution 3 M dans le diéthyléther de bromure de phénylmagnésium maintenue vers 0°C, on coule en 1 heure, une solution de 22 g (0,1325 mole) de 4-hexaméthylèneimino-butyronitrile dans 50 ml de diéthyléther. On laisse reposer le milieu réactionnel pendant une nuit à la température ambiante (15-20°C) et on jette ledit milieu réactionnel sur 220 ml d'eau glacée et 110 ml d'acide chlorhydrique 12 N. On agite 1 h, on isole le précipité formé par filtration et on le remet en suspension dans l'eau. Après alcalinisation par de la soude concentrée et extraction par le diéthyléther, on obtient 26 g d'une huile légèrement jaune. Cette huile est traitée dans le diéthyléther au moyen d'éthanol chlorhydrique. On recueille le précipité formé. Par recristallisation de ce dernier dans l'éthanol anhydre on obtient 25,8 g (rendement: 69,2%) de CRL 41 339 qui se présente sous la forme d'une poudre blanche très soluble dans l'eau (100 g/l). $F_{inst}$ = 192°C.

On a résumé ci-après les résultats des essais pharmacologiques qui ont été entrepris avec le CRL 41 399.

I. *Toxicité*

Par voie intrapéritonéale chez la souris on constate que la DL-O (dose maximale non mortelle) est supérieure à 64 mg/kg, que la DL-30 (dose provoquant la mort de 30% des animaux) est de l'ordre de 128 mg/kg, et que la DL-100 (dose minimale provoquant la mort de tous les animaux) est inférieure ou égale à 256 mg/kg.

II. *Etude cardiovasculaire*

L'étude des propriétés cardiovasculaires a été réalisée chez le chien anesthésié au Nembutal®, le CRL 41 339 étant administré par voie intraduodénale en solution dans du sérum physiologique (eau bidistillée renfermant 9 g/l de NaCl) à pH 7,5 (la concentration maximale utilisée étant de 32,4 mg/ml de CRL 41 339).

Trois chiens (poids moyen 14,3 kg) anesthésiés au Nembutal® reçoivent le CRL 41 339 par voie I.D. aux doses successives de 0,1 mg/kg, 0,5 mg/kg, 1 mg/kg, 2,5 mg/kg, 5 mg/kg et 10 mg/kg, deux de ces chiens recevant ensuite une dose supplémentaire par voie intraveineuse de 10 mg/kg de CRL 41 339.

On mesure la pression artérielle, la fréquence cardiaque, le débit artériel fémoral, le débit artériel vertébral, les températures rectale et cutanée.

On constate que le CRL 41 339, administré par voie I.D., augmente de façon importante (à partir de 2,5 mg/kg — c'est-à-dire à la dose cumulée de 4,1 mg/kg-) le débit artériel fémoral, qu'il est sans action sur la pression artérielle et la fréquence cardiaque, et qu'il ralentit le rythme respiratoire, les températures rectale et cutanée n'étant pas modifiées.

La dose supplémentaire de 10 mg/kg I.V. de CRL 41 339 entraîne une hypotension et une bradycardie.

Les effets de l'isoprénaline déterminés après administration de la dose cumulée de 19,1 mg/kg I.D. de CRL 41 339 sont légèrement diminués en ce qui concerne la fréquence cardiaque et non modifiés en ce qui concerne la pression artérielle diastolique: après administration de 1 µg/kg d'isoprénaline, on constate que le CRL 41 399 fait passer (i) la pression artérielle diastolique de 117 mmHg (soit environ $1,5 \times 10^4$ Pa) à 37 mmHg (soit environ $4,9 \times 10^3$ Pa) alors que celle des témoins (chiens anesthésiés ne recevant que l'isoprénaline) passe de 144 mmHg (soit environ $1,9 \times 10^4$ Pa) à 36 mmHg (soit environ $4,8 \times 10^3$ Pa), et (ii) la fréquence cardiaque de 175 battements/minute à 238 battements/minute, alors que celle des témoins passe de 188 battements/minute à 253 battements/minute.

On constate également que l'hypertension induite par la noradrénaline est diminuée par le CRL 41 399 (à la dose cumulée de 19,1 mg/kg I.D.): après administration de 2 µg/kg de noradrénaline, le CRL 41 399 fait passer la pression artérielle systolique de 160 mmHg (soit environ $2,1 \times 10^4$ Pa) à 253 mmHg (soit environ $3,3 \times 10^4$ Pa) alors que celle de témoins (chiens anesthésiés ne recevant que la noradrénaline) passe de 181 mmHg (soit environ $2,4 \times 10^4$ Pa) à 301 mmHg (soit environ $4 \times 10^4$ Pa).

III. *Essais comparatifs*

Le CLR 41 399 selon l'invention a été comparé à des composés analogues (i.e. le composé de référence A-1, et le composé A-3) et à un composé homologue (composé A-2). Les résultats consignés dans le tableau I ci-après mettent en évidence la supériorité du CLR 41 399 (Ex. 1), par rapport aux produits structurellement voisins antérieurement connus, en ce qui concerne la toxicité et l'activité vasodilatative périphérique.

IV. *Essais cliniques*

En clinique, chez l'homme adulte, on a obtenu de bons résultats avec le CRL 41 399 en tant que vasodilatateur notamment dans le traitement (i) des manifestations de l'artérite des membres inférieurs et (ii) des ulcères d'origine arterielle ou veineuse des membres inférieurs.

La posologie que l'on préconise consiste à administrer à l'homme des gélules ou comprimés renfermant chacun 100 mg de CRL 41 339, à raison de 2 à 3 comprimés ou gélules par jour.

TABLEAU I

AR-CO-$(CH_2)_3$-B, HCl

| Produit | No. de Code | Ar | B | DL-50 I.V. souris (mg/kg) | DMV (e) (mg/kg) | Index thérapeutique (h) |
|---|---|---|---|---|---|---|
| Ex 1 (a) | CRL 41 339 | $C_6H_5$ | hexaméthylène-imino | >128 (f) | ≤4,1 | 0,032 |
| A-1 (b) (c) | LL 1656 | 2,4,6-$(OCH_3)_3C_6H_2$ | pyrrolidino | 60 | 6 | 0,100 |
| A-2 (d) | CRL 31 080 | 2,4,6-$(OCH_3)_3C_6H_2$ | hexaméthylène-imino | 96 | 5 | 0,053 |
| A-3 (c) | LL 1647 | 2,4,6-$(OCH_3)_3C_6H_2$ | pipéridino | 68 | (g) | 0 |

*Notes:* (a) produit selon l'invention;
(b) vasodilatateur de référence;
(c) décrit dans US-A-3 895 030;
(d) décrit dans FR-A-2 534 916;
(e) dose minimale induisant chez le chien anesthésié au Nembutal® une action vaso-dilatatrice fémorale (les produits étudiés étant administrés par voie I.D.);
(f) 128 mg/kg est approximativement la DL-30 I.V. du CRL 41 339;
(g) pas d'action vasodilalatrice par voie I.D.;
(h) rapport DmV/DL-50

V. *Essais complémentaires*

On a résumé ci-après les résultats des essais complémentaires qui ont été entrepris avec le CRL 41 399.

a) *Activité mutagène.*

On constate que le CRL est dépourvu d'effets mutagènes, notamment sur *Salmonella typhimurium*.

b) *Action sur la microcirculation de l'oreille de lapin.*

On administre par voie gastrique le CRL 41 399 en solution aqueuse à pH de 6,5 environ préparée extemporanément à des lots de lapins Half Top (3 mâles d'un poids moyen de 3,5 kg, et 3 femelles d'un poids moyen de 3,3 kg) sous un volume de 2 ml/kg. Les lapins utilisés comme témoins par rapport à eux-mêmes reçoivent de l'eau distillée par voie gastrique, sous le même volume, à titre de contrôle. On mesure les paramètres suivants: diamètre artériolaire terminal, diamètre veinulaire, pulsatilité artériolaire, température cutanée de l'oreille, fréquence cardiaque et température rectale.

On constate que chez le lapin Half Top éveillé, le CRL 41 339 administré par voie gastrique à la dose de 10 mg/kg, dilate modérément pendant 0,5 h l'artériole terminale, augmente fortement la pulsatilité artériolaire et le rapport pulsatilité artériolaire/fréquence cardiaque sans modifier la fréquence cardiaque, entraîne une élévation importante et durable de la température cutanée de l'oreille, et n'a aucune action sur le diamètre veinulaire.

c) *Action sur les vaisseaux isolés de chien.*

Des anneaux d'artères fémorales et de veines saphènes externes de chiens anesthésiés au Nembutal® sont prélevés et placés dans du Krebs-Henseleit aéré ($O_2$ : 96%, $CO_2$ : 4%, en volume) maintenu à 37°C, et, soumis à une tension de 0,5 g. Ces vaisseaux sont contractés soit par la noradrénaline $10^{-5}$M en présence de propanolol $10^{-6}$M, soit par le KCl 50 mM en présence de phentolamine $3 \times 10^{-6}$M. Le CRL 41 339 de $10^{-6}$M à $3 \times 10^{-4}$M est étudié en dose cumulées.

On constate que le CLR 41 339 est dépourvu d'effet sur la contraction de l'artère (n = 5) et de la veine (n = 4) par le KCl en présence de phentolamine, et, qu'il relâche l'artère (n = 5) et la veine (n = 4) contractées par la noradrénaline en présence de propanolol, les concentrations molaires (CI-50) du CRL 41 339 qui diminuent la contraction de 50% étant alors les suivantes:

— sur l'artère, CI-50 = $(5,9 \pm 3,09) \times 10^{-6}$M
— sur la veine, CI-50 = $(1,3 \pm 0,68) \times 10^{-4}$M

d) *Action sur le train postérieur perfusé de rat.*

Chez le rat anésthésié au Nembutal®, le CRL 41 399 présente un effet vasodilatateur important sur le train postérieur perfusé contracté au moyen de noradrénaline. La durée de cet effet augmente avec la dose de CRL 41 339. On présume que l'activité anti-noradrénaline importante du CRL 41 339 participe à l'activité vasodilatatrice dudit CRL 41 339.

e) *Comparaison de differents sels.*

Trois chiens anesthésiés au Nembutal® ayant un poids moyen de 12,9 kg sont utilisés comme témoins par rapport à eux-mêmes et reçoivent le chlorhydrate, le fumarate et le citrate de phényl-(3-hexaméthylèneiminopropyl)-cétone (numéros de code respectifs: CRL 41 339, CRL 41 339 A et CRL 41 339 B) par voie intra-artérielle fémorale, à doses équimolaires.

On constate que les quantités de produits injectées ne modifient pas la pression artérielle ni la fréquence cardiaque. En revanche les trois produits possèdent une action vasodilatatrice fémorale importante comme le montrent les résultats du tableau II ci-après relatifs au débit fémoral (moyenne de 5 essais par produit et par dose).

TABLEAU II

| Produit | N° de code | dose | Débit Fémoral (ml/min) |
|---|---|---|---|
| Ex 1 (a) | CRL 41 339 | 0 | 64 |
| Ex 1 (a) | CRL 41 339 | $10^{-7}$M | 83 |
| Ex 1 (a) | CRL 41 339 | $10^{-6}$M | 117 |
| Ex 1 (a) | CRL 41 339 | $10^{-5}$M | 166 |
| Ex 2 (b) | CRL 41 339A | 0 | 64 |
| Ex 2 (b) | CRL 41 339A | $10^{-7}$M | 76 |
| Ex 2 (b) | CRL 41 339A | $10^{-6}$M | 117 |
| Ex 2 (b) | CRL 41 339A | $10^{-5}$M | 168 |
| Ex 3 (c) | CRL 41 339B | 0 | 79 |
| Ex 3 (c) | CRL 41 339B | $10^{-7}$M | 90 |
| Ex 3 (c) | CRL 41 339B | $10^{-6}$M | 127 |
| Ex 3 (c) | CRL 41 339B | $10^{-5}$M | 197 |

*Notes:* (a): Chlorhydrate de phényl-(3-hexaméthylèneiminopropyl)-cétone; pH d'administration par voie I.A.: pH 6;
(b): fumarate de phényl-(3-hexyméthylèneiminopropyl)-cétone; pH d'administration par voie I.A.: pH 4;
(c): citrate de phényl-(3-hexaméthylèneiminopropyl)-cétone; pH d'administration par voie I.A.: pH 4.

**Revendications pour les Etats contractants:**
BE, CH, DE, FR, GB, IT, LI, LU, NL, SE

1. Dérivé de phényl-aminoalkyl-cétone, caractérisé en ce qu'il est choisi parmi l'ensemble constitué par

a) la phényl-(3-hexaméthylèneiminopropyl)-cétone de formule

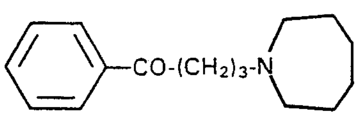

(I)

et

b) ses sels d'addition.

2. Chlorhydrate de phényl-(3-hexaméthylèneiminopropyl)-cétone.

3. Composition thérapeutique, caractérisée en ce qu'elle renferme, en association avec un récipient physiologiquement acceptable, au moins un dérivé choisi parmi l'ensemble constitué par la phényl-(3-hexaméthylèneiminopropyl)-cétone, et ses sels d'addition non-toxiques.

4. Utilisation d'une substance choisie parmi la phényl-(3-hexaméthylèneiminopropyl)-cétone et ses sels d'addition non-toxiques pour l'obtention d'un médicament vasodilatateur destiné aux patients atteints de troubles circulatoires notamment des membres inférieurs.

5. Procédé de préparation de la phényl-(3-hexaméthylèneiminopropyl)-cétone et de ses sels d'addition, caractérisé en ce qu'il comprend la réaction du 4-hexaméthylèneimino-butyronitrile avec le bromure de phénylmagnésium pendant au moins 1 h à une température comprise entre 0 et 25°C dans un solvant anhydre convenable, à raison de plus d'une mole de bromure de phénylmagnésium pour une mole de 4-hexaméthylèneimino-butyronitrile.

**Revendication pour l'Etat contractant:** AT

1. Procédé de préparation de la phényl-(3-hexaméthylèneiminopropyl)-cétone et de ses sels d'addition, caractérisé en ce qu'il comprend la réaction du 4-hexaméthylèneimino-butyronitrile avec le bromure de phénylmagnésium pendant au moins 1 h à une température comprise entre 0 et 25°C dans un solvant anhydre convenable, à raison de plus d'une mole de bromure de phénylmagnésium pour une mole de 4-hexaméthylèneimino-butyronitrile.

**Patentansprüche für die Vertragsstaaten:**
BE, CH, DE, FR, GB, IT, LI, LU, NL, SE

1. Phenylaminoalkylketon-Derivat, dadurch gekennzeichnet, daß es aus der Gruppe ausgewählt ist, die

a) Phenyl-(3-hexamethyleniminopropyl)-keton der Formel

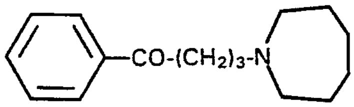

(I)

und

b) dessen Additionssalze umfaßt.

2. Hydrochlorid des Phenyl-(3-hexamethyleniminopropyl)-ketons.

3. Therapeutische Zubereitung, dadurch gekennzeichnet, daß sie mindestens ein Derivat ausgewählt aus der Phenyl-(3-hexamethyleniminopropyl)-keton und dessen nichttoxische Additionssalze umfassenden Gruppe in Kombination mit einem physiologisch annehmbaren Träger enthält.

4. Verwendung einer aus Phenyl-(3-hexamethyleniminopropyl)-keton und dessen nichttoxischen Additionssalzen ausgewählten Substanz zur Herstellung eines Arzneimittels mit vasodilatatorischer Wirkung zur Behandlung von Patienten mit Kreislaufstörungen, insbesondere im Bereich der unteren Gliedmaßen.

5. Verfahren zur Herstellung von Phenyl-(3-hexamethyleniminopropyl)-keton und dessen Additionssalzen, dadurch gekennzeichnet, daß es die Umsetzung von 4-Hexamethylenimino-butyronitril mit Phenylmagnesiumbromid während mindestens einer Stunde bei einer Temperatur zwischen 0 und 25°C in einem geeigneten wasserfreien Medium umfaßt, wobei mehr als ein Mol Phenylmagnesiumbromid pro Mol 4-Hexamethyleniminobutyronitril eingesetzt werden.

**Patentanspruch für den Vertragsstaat:** AT

1. Verfahren zur Herstellung von Phenyl-(3-hexamethyleniminopropyl)-keton und dessen Additionssalzen, dadurch gekennzeichnet, daß es die Umsetzung von 4-Hexamethylenimino-butyronitril mit Phenylmagnesiumbromid während mindestens einer Stunde bei einer Temperatur zwischen 0 und 25°C in einem geeigneten wasserfreien Medium umfaßt, wobei mehr als ein Mol Phenylmagnesiumbromid pro Mol 4-Hexamethyleniminobutyronitril eingesetzt werden.

**Claims for the Contracting States:**
BE, CH, DE, FR, GB, IT, LI, LU, NL, SE

1. Derivative of phenyl-aminoalkyl-ketone, characterised in that it is selected among the set constituted by

a) phenyl-(3-hexamethylene iminopropyl)-ketone of formula

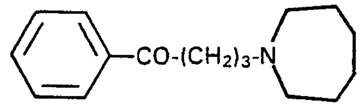

(I)

and

b) its salts of addition.

2. Phenyl-(3-hexamethylene iminopropyl)-ketone hydrochloride.

3. Therapeutical compound, characterized in that it contains, in association with a physiologically acceptable recipient, at least one derivative selected among the set constituted by phenyl-(3-hexamethylene iminopropyl)-ketone and its non-toxic salts of addition.

4. Utilisation of a substance selected among phenyl-(3-hexamethylene iminopropyl)-ketone and its non-toxic salts of addition for the obtaining of a vaso-dilator medicament intended for patients affected by circulatory troubles, especially of the lower members.

5. Process for preparation of phenyl-(3-hexamethylene iminopropyl)-ketone and its salts of addition, characterised in that it comprises the reaction of 4-hexamethylene iminobutyronitrile with phenyl magnesium bromide for at least 1 h at a temperature between 0 and 25°C in a suitable anhydrous solvent, at the rate of more than one mol of phenyl magnesium bromide for one mol of 4-hexamethylene iminobutyronitrile.

**Claim for the Contracting State:** AT

1. Process for preparation of phenyl-(3-hexamethylene iminopropyl)-ketone and its salts of addition, characterised in that it comprises the reaction of 4-hexamethylene iminobutyronitrile with phenyl magnesium bromide for at least 1 h at a temperature between 0 and 25°C in a suitable anhydrous solvent, at the rate of more than one mol of phenyl magnesium bromide for one mol of 4-hexamethylene iminobutyronitrile.